Europäisches Patentamt

European Patent Office

Office européen des brevets

(11) Publication number: **0 089 187**
**B1**

(12) **EUROPEAN PATENT SPECIFICATION**

(45) Date of publication of patent specification: **20.09.89**

(21) Application number: **83301329.5**

(22) Date of filing: **10.03.83**

(51) Int. Cl.⁴: **A 61 K 6/08, C 09 D 3/74**

(54) **Varnish for protecting a tooth surface.**

(30) Priority: **12.03.82 JP 39694/82**

(43) Date of publication of application:
**21.09.83 Bulletin 83/38**

(45) Publication of the grant of the patent:
**20.09.89 Bulletin 89/38**

(84) Designated Contracting States:
**DE FR GB NL**

(56) References cited:
**FR-A-2 364 660**
**US-A-3 507 822**

**CHEMICAL ABSTRACTS, vol. 97, 27 December 1982, page 448, abstract 222999z (COLUMBUS, OHIO, US) & JP-A-82 130 907 (SANKIN INDUSTRIES) 13 August 1982**

(73) Proprietor: **KURARAY CO., LTD.**
**1621 Sakazu**
**Kurashiki-City Okayama Prefecture 710 (JP)**

(72) Inventor: **Fusayama, Takao**
**4-16-23, Kamiigusa**
**Suginami-ku Tokyo-city (JP)**
Inventor: **Yamauchi, Junichi**
**21-7, Hachioji-cho**
**Kurashiki-city (JP)**

(74) Representative: **Crampton, Keith John Allen et al**
**D YOUNG & CO 10 Staple Inn**
**London WC1V 7RD (GB)**

Courier Press, Leamington Spa, England.

## EP 0 089 187 B1

**Description**

This invention is concerned with the protection of healthy tooth surfaces against erosion by an acid etching agent in tooth restoration treatment and with a varnish for forming a temporary coating on a tooth surface.

Hitherto teeth have often been restored by removing a carious part and filling a composite resin into it to restore the tooth. In this method, the adhesion between the tooth and the composite resin becomes a problem, and if the adhesion is poor, the filling falls out or a further caries develops between the tooth and the filling. For that reason, it is necessary to enhance the adhesion. This is commonly done by treating the tooth surface with an acid etching agent comprising an aqueous solution, e.g. of phosphoric acid or citric acid, and further coating with a bonding agent containing an adhesive monomer before filling the composite resin [J. Dent. Res., *34*, 849 (1955), US Patent No. US—A—4,259,075]. Although the acid etching agent is effective for imparting adhesion between the tooth and the composite resin by giving minute unevenness to the tooth surface, it has the disadvantage that if the treating agent spreads over to a healthy tooth surface, it also etches the healthy tooth surface (especially the enamel) to cause unevenness, where dental plaque is generated and bacteria cling, thus leading to the formation of a caries. In order to prevent such a situation, attempts have also been made to increase the viscosity of the acid etching agent, e.g. by adding carboxymethyl cellulose or highly dispersed submicron silica to it, thereby preventing the spreading of the acid (Japanese Patent Application Laid open No. JP—A—36994/1978), but it is difficult to apply the acid etching agent only to the carious part and therefore adhesion of the acid etching agent to a part of the healthy tooth surface is unavoidable.

By applying a varnish comprising a polymer solution to a tooth surface to form a polymer coating on the tooth surface before applying an acid etching agent in conventional manner to the surface of a tooth cavity, and subsequently removing the polymer coating from the tooth surface, the erosion of a healthy tooth surface by an acid etching agent can be prevented.

US Patent Specification US—A—3,507,822 discloses coating compositions comprising a cyanoacrylate monomer composition and a vinyl polymer, e.g. a copolymer (a) of 60% vinyl chloride, 30% vinyl acetate and 10% methyl methacrylate, (b) of 75% vinyl chloride and 25% vinyl acetate or (c) of 70% vinyl chloride and 30% vinyl acetate. Polymer (a) is said to be particularly suitable for dental use.

In accordance with the present invention, there is provided a varnish comprising a polymer which has vinyl chloride and vinyl acetate as main constituent monomer units and which is dissolved in a solvent, the varnish being suitable for use in the formation of a coating on a tooth surface for protecting a healthy tooth surface against erosion by an acid etching solution in tooth restoration treatment, in which the polymer comprises a mixture of a copolymer (A) of vinyl chloride and vinyl acetate and a copolymer (B) of vinyl chloride, vinyl acetate and a vinyl compound having a carboxylic acid or carboxylic anhydride group or groups, the molar ratio of vinyl chloride to vinyl acetate in copolymers (A) and (B) being (95 to 60):(5 to 40) and the molar proportion of the vinyl compound having a carboxylic acid or carboxylic anhydride group or groups to the sum of the vinyl chloride and vinyl acetate in copolymer (B) being 1 to 20%.

In this invention, the polymer coating formed on a tooth surface must be stable, i.e. properly stuck to the tooth (so that when an acid etching agent is applied to the surface of a tooth cavity from which the carious part has been removed, the acid etching agent does not spread over the healthy surface of the tooth) and at the same time easily cut when the carious part is removed by a drilling operation, so that only the coating on the carious surface is removed and the coating on the healthy tooth surface remains intact. Further, the polymer coating on the tooth surface is desirably removable simply by polishing. Therefore, as the polymer solution to be applied to a tooth surface in this invention, a solution containing a polymer having the above-described characteristics and harmless to the human body is used. The carboxylic acid or carboxylic acid anhydride groups improve tackiness to the enamel of a tooth.

Examples of copolymers (A) and (B) used in the compositions of the present invention are known polymers used in paints and coating materials for application to plastics, metals and cements. Such polymers are described in, for example, Japanese Patent Publication No. JP—B—17505/1968 Japanese Patent Application Laid-open No. JP—A—61235/1979 and Canadian Patent CA—A—745,215. Of course, the use of such polymers for the purpose of this invention is not disclosed in any of the above literature.

If the content of the vinyl chloride component exceeds 95 mole %, the tackiness of the varnish to the enamel is poor, whereas if the vinyl acetate component is more than 40 mole %, removal of the coating becomes poor when the carious part is removed using a bar, as described hereinbelow, after coating the tooth surface, and thus this is not preferred. The ratio of the vinyl chloride to vinyl acetate in the copolymers (A) and (B) is preferably (90—72):(10—28) mole %. In copolymer (B), the vinyl compound having a carboxylic acid or carboxylic anhydride group or groups preferably makes up 2—10 mole % based on the vinyl chloride plus vinyl acetate components. By the presence of an appropriate amount of the said carboxylic acid or anhydride group or groups, the tackiness to the enamel is enhanced. Examples of such vinyl compounds include acrylic acid, methacrylic acid, maleic acid, fumaric acid, itaconic acid and crotonic acid, and their acid anhydrides.

The proportion of the copolymer (A) to the copolymer (B) is preferably (95—40):(5—60)% by weight, particularly (90—60):(10—40)% by weight. If the amount of the copolymer (B) is too great, there is a tendency for the coating to become hard and brittle.

Both copolymers (A) and (B) preferably have a molecular weight in the range of 15,000—150,000 (particularly 20,000—70,000). If the molecular weight is too small, the strength of the coat is low, whereas if the molecular weight is too great, the tackiness to the tooth surface is reduced. Furthermore, the copolymers used in this invention may contain vinyl alcohol formed by the hydrolysis of a part of the vinyl acetate and may also contain small amounts (up to 10% by weight per 100 parts by weight of the vinyl chloride plus vinyl acetate units) of vinyl esters other than vinyl acetate.

Both copolymers (A) and (B) preferably have a molecular weight in the range of 15,000—150,000 (particularly 20,000—70,000). If the molecular weight is too small, the strength of the coat is low, whereas if the molecular weight is too great, the tackiness to the tooth surface is reduced. Furthermore, the copolymers used in this invention may contain vinyl alcohol formed by the hydrolysis of a part of the vinyl acetate and may also contain small amounts (up to 10% by weight per 100 parts by weight of the vinyl chloride plus vinyl acetate units) of vinyl esters other than vinyl acetate.

According to this invention, the aforesaid polymer is applied in solution in an organic solvent. As the organic solvent, one having a boiling point of 140°C or below and substantially harmless to the human body is used. Examples of such solvents include acetone, methyl ethyl ketone, diethyl ether, diisopropyl ether, ethanol, n-propanol, isopropanol, tetrahydrofuran, methyl acetate, ethyl acetate, n-propyl acetate, isopropyl acetate, n-butyl acetate, isobutyl acetate, methylene chloride, ethylene chloride, chloroform, carbon tetrachloride, 1,1,1-trichloroethane, 1,1,2-trichloroethane, 1,1,1,2-tetrachloroethane, 1,1,2,2-tetrachloroethane, cyclohexane, toluene, ethylene glycol monomethyl ether, and ethylene glycol monoethyl ether, and they may be used either alone or as a mixture of two or more of them. Among those, acetone, diethyl ether, diisopropyl ether, ethanol, ethyl acetate, n-propyl acetate, isopropyl acetate, n-butyl acetate, isobutyl acetate, methylene chloride, ethylene chloride and chloroform are preferred. The polymer is dissolved in the solvent to a concentration of 1—20% by weight (preferably 5—15% by weight) and applied to a tooth surface.

In this invention, the varnish also preferably contains a silane coupling agent having affinity with the hydroxyapatite on a tooth surface in order to further enhance the tackiness between the polymer and the tooth surface. Examples of such silane coupling agents include di- and trialkoxysilane compounds such as vinyltriethoxysilane, vinyltris(β - methoxyethoxy)silane, γ - glycidoxypropyltrimethoxysilane, γ - methacryloxypropyltrimethoxysilane, N - β(aminoethyl) -γ - aminopropyltrimethoxysilane, N - β(amino- ethyl) - γ - aminopropylmethyldimethoxysilane, γ - ureidopropyltriethoxysilane, γ - chloropropyl- trimethoxysilane, γ - mercaptopropyltrimethoxysilane, and β - (3,4 - epoxycyclohexyl)ethyl - trimethoxysilane. The amount of silane coupling agent added is preferably up to 20% by weight (particularly 3—10% by weight) based on the polymer. If the amount of the silane coupling agent added is too great, the strength of the coating is reduced.

In this invention, it is preferred to further add a dye to the varnish to colour it. This has a merit in tooth restoration treatment, that is, after the varnish has been applied to a tooth surface, the carious part is removed and a composite resin is filled into the resulting gap; if the varnish is coloured, it clearly indicates the boundary between the protected tooth surface (healthy part) and the part to be filled and facilitates the shaping of the filled-in resin in conformity to the gap (for example, by removing the excess of resin). As such a dye, those substantially harmless to the human body are preferred, and cosmetic dyes that are sparingly soluble in water and soluble in organic solvents are preferably used. Examples of such dyes include Sudan III (Red No. 225), Rhodamine B Acetate (Red No. 214), Indigo (Blue No. 201), Sudan Blue B (Blue No. 403), and Phthalocyanine Blue (Blue No. 404). In addition, inorganic pigments such as carbon black, iron oxide and chromium oxide may be used. The amount of dye added to the varnish is 0.01—2% by weight (based on the total liquid weight).

By using the varnish for tooth protection composed described above, restoration of a tooth is conducted as follows: First, the varnish of this invention is applied to a surface of a tooth requiring restoration treatment and the solvent is evaporated to form a coat of a polymer on the tooth surface. The evaporation of the solvent is preferably achieved by blowing air, and the thickness of the coat formed on the tooth surface is generally 1—10 µm or so. Thereafter, the carious part is removed by a drill, and then the tooth surface of the part to be filled is treated with an acid etching agent. At that time, since the healthy tooth surface is covered with the varnish of this invention, the acid etching agent does not spread to healthy tooth surface. After the acid etching treatment, the carious surface to be further filled is coated with a bonding agent containing an adhesive monomer, and thereafter a composite resin is filled into it. In such a case, the composite resin must be formed in the shape according to the tooth shape (in particular for a molar tooth); if the varnish is coloured, the boundary between the tooth and the filled resin is clearly indicated and thus this is very convenient in tooth restoration treatment. After completion of the restoration, the varnish is scraped off by an appropriate dental tool. Alternatively, it is also possible to remove the carious part by a drill and then coat the healthy tooth surface with the varnish (this must be conducted carefully lest the varnish enters into the carious cavity) and thereafter treat the surface with the caries with the acid etching agent.

In the above restoration of a tooth, the removal of the caries part using a drill, the acid etching treatment, the application of the bonding agent and the filling of the dental filling material are conducted in conventional manner. Examples of ths acid etching agents to be used are described in the aforesaid J. Dent. Res., *34,* 849 (1955) and Japanese Patent Application Laid-open No. JP—A—36994/1978, the bonding agent to be used is exemplified in e.g. US Patents US—A—4,148,988, 4,182,035, 4,222,780, 4,259,075, 4,259,117 etc., and as the dental filling material, those described in US Patents US—A—3,926,906, 4,302,381, 4,347,174 etc. may be used.

As described hereinabove, since the varnish for protecting a tooth surface which comprises a vinyl chloride—vinyl acetate copolymer has not only excellent tackiness to the tooth surface consisting of enamel but also appropriate strength, by using such a varnish in tooth restoration treatment, the healthy tooth surface can be protected from the erosion by an acid etching agent even when removing the carious part by a drill after covering the tooth surface and then conducting the acid etching treatment, and therefore this invention greatly contributes to dental treatment.

Illustrative and non-limiting examples of this invention are given below. Various modifications are possible based on the idea of this invention.

Example 1

Varnishes having the respective compositions set forth in Table 1 were prepared, and tackiness of each to a tooth surface was evaluated by applying to a human extracted tooth. The application to the tooth surface was conducted as follows: The tooth surface was cleaned with ethanol, dried by blowing air, and each varnish impregnated in a sponge was applied to the surface. The solvent was evaporated using an air gun, and a tooth cavity (the size of the cavity: 3—4 mm in diameter) was formed in the centre of the tooth surface covered with the coat using an air turbine (using a carbide bar). The tackiness of the coat to the tooth surface after the tooth cavity formation was evaluated by visual observation and also by the degree of penetration into the dyed coat of a drop of Indian ink dropped onto the tooth surface. The results are given in Table 1.

TABLE 1

| No. | 1 | 2 | 3 | 4 | 5 | 6 | 7 | Comparative |
|---|---|---|---|---|---|---|---|---|
| Composition (parts by weight) | | | | | | | | |
| Polymer (A) | 10.0 | 9.5 | 8.5 | 9.0 | 8.5 | 8.0 | 8.0 | — |
| Polymer (B) | — | — | 1.5 | 0.5 | 1.5 | 2.0 | 1.0 | — |
| Polymer (C) | — | — | — | — | — | — | — | 10.0 |
| Silane treating agent (M) | — | — | — | 0.5 | 0.5 | — | — | — |
| Silane treating agent (N) | — | 0.5 | — | — | — | 0.5 | 1.0 | — |
| Solvent (X) | 90.0 | — | 90.0 | 90.0 | 89.5 | 89.5 | — | — |
| Solvent (Y) | — | 90.0 | — | — | — | — | 90.0 | — |
| Solvent (Z) | — | — | — | — | — | — | — | 90.0 |
| Tackiness | A | A | AA | AA | AAA | AAA | AAA | B |

Note 1) Respective components
    Polymer (A): Vinyl chloride—vinyl acetate (80:20 mole %) copolymer, M.W. 60,000
    Polymer (B): Vinyl chloride—vinyl acetate—maleic anhydride (85:10:5 mole %) copolymer, M.W. 50,000
    Polymer (C): Polyvinyl chloride
    Silane treating agent (M): γ-Methacryloxypropyltrimethoxysilane
    Silane treating agent (N): Vinyltriethoxysilane
    Solvent (X): Ethyl acetate—n-butyl acetate (80:20% by weight)
    Solvent (Y): Ethyl acetate—acetone (70:30% by weight)
    Solvent (Z): Dichloromethane

Note 2) Evaluation of Tackiness
    AAA: The tackiness to the tooth surface is very good. (The condition of the coat after the tooth cavity formation is good).
    AA: The tackiness to the tooth surface is good. (The condition of the coat after the tooth cavity formation is good).
    A: The tackiness to the tooth surface is moderate. (A part of the coat has come off the tooth surface, but the rest remains stuck to the tooth surface).
    B: The tackiness to the tooth surface is poor. (The greater part of the coat has come off the tooth surface).
    As evident from Table 1, with the varnishes of this invention, tackiness to the tooth surface was observed. Especially when the polymer containing maleic anhydride as a component was used in combination, and further where the silane coupling agents were added, excellent tackiness to the tooth surface was exhibited.

Example 2
    Using that obtained by adding 0.5% by weight (based on the total liquid weight) of Phthalocyanine Blue dye to the varnish referred to as No. 5 in Table 1, tooth restoration treatment was conducted as follows: A piece of sponge impregnated with the varnish was applied to the surface of a molar tooth having a caries. The tooth surface was dried using an air gun to form a coat. After the formation of the coat, the carious part was removed using a drill while being guided by a caries detector disclosed in US Patent No. US—A—4,347,233 to form a class 2 cavity. The coating stuck to the healthy tooth surface was stable even after the formation of the cavity. Thereafter, a 40% phosphoric acid aqueous solution was applied to the wall of the cavity and dried, after which the wall of the cavity was further applied with a bonding agent (commercial name: Clearfill Posterior) and dried. Thereafter, a composite resin of Clearfil Posterior was filled into the tooth cavity along the outer line of the coloured coating on the tooth crown and hardened. Thus, the carious tooth was treated and since the tooth surface was protected by the varnish of this invention, the spreading of the acid etching agent over to the healthy tooth surface was not observed. Further, the composite resin was overflowed from the tooth cavity at the time of restoring the tooth must be removed from the tooth surface, but since the coat was coloured, it was easy to distinguish between the tooth surface and the composite resin, and it was possible to easily remove the excess of resin. After restoration, it was possible to remove the coloured coat by scraping.

Example 3

An enamel caries showing a slight chalk colour on a molar occlusal surface was cut and removed using a drill to form a tooth cavity. Thereafter, a varnish having the composition referred to as No. 6 in Table 1 and coloured by further adding to it 0.5% by weight (based on the total liquid weight) of Phthalocyanine Blue dye was applied to the periphery of the tooth cavity using a piece of sponge carefully, to prevent the varnish from running into the inner part of the cavity, and the tooth surface was gently dried using an air gun to form a coat. After confirming that the coat did not sag into the inner part of the cavity, a 40% phosphoric acid aqueous solution was applied to the entire tooth cavity, then bonding agent was applied to the cavity surface and dried. Thereafter, a composite resin was filled into the tooth cavity and hardened. The resin that overflowed from the tooth cavity was scraped off using a finish polishing bar, using the blue-coloured coating present beneath the resin as a guide until the blue coat had been completely removed. Thus, where the colouration of the caries was small, as in this case, by applying the varnish after forming the tooth cavity, restoration was successfully conducted while protecting the healthy enamel against the acid.

**Claims**

1. A varnish comprising a polymer which has vinyl chloride and vinyl acetate as main constituent monomer units and which is dissolved in a solvent, the varnish being suitable for use in the formation of a coating on a tooth surface for protecting a healthy tooth surface against erosion by an acid etching solution in tooth restoration treatment, characterized in that the polymer comprises a mixture of a copolymer (A) of vinyl chloride and vinyl acetate and a copolymer (B) of vinyl chloride, vinyl acetate and a vinyl compound having a carboxylic acid or carboxylic anhydride group or groups, the molar ratio of vinyl chloride to vinyl acetate in copolymers (A) and (B) being (95 to 60):(5 to 40) and the molar proportion of the vinyl compound having a carboxylic acid or carboxylic anhydride group or groups to the sum of the vinyl chloride and vinyl acetate in copolymer (B) being 1 to 20%.

2. A varnish according to Claim 1 in which the proportion by weight of copolymer (A) to copolymer (B) is in the range (95 to 40):(5 to 60)

3. A varnish according to Claim 2 in which the said proportion is in the range (90 to 60):(10 to 40).

4. A varnish according to any one of Claims 1 to 3 in which the molar ratio of vinyl chloride to vinyl acetate in copolymers (A) and (B) is (90 to 72):(10 to 28).

5. A varnish according to any one of Claims 1 to 4 that further contains 3 to 10% by weight (based on the polymer) of a silane coupling agent so as to enhance the tackiness of the tooth-surface-protecting components to the tooth surface.

6. A varnish according to any one of Claims 1 to 5 that further contains 0.01 to 2% by weight (based on the solution) of a dye so as to indicate the boundary between the part of the tooth surface to be protected and the part of it to be restored.

7. A varnish according to any one of Claims 1 to 6 in which the concentration of total copolymers is 1 to 20% by weight, based on the solution.

8. A varnish according to any one of Claims 1 to 7 in which the solvent is acetone, diethyl ether, diisopropyl ether, ethanol, ethyl acetate, n-propyl acetate, isopropyl acetate, n-butyl acetate, isobutyl acetate, methylene chloride, ethylene chloride or chloroform or a mixture of two or more such compounds.

9. A varnish according to any one of Claims 1 to 8 in which each of the copolymers (A) and (B) has a molecular weight in the range 20,000 to 70,000.

**Patentansprüche**

1. Lack aus einem Polymeren, das Vinylchlorid und Vinylacetat als Hauptmonomereinheiten enthält und in einem Lösungsmittel gelöst ist, wobei der Lack geeignet ist für die Bildung eines Überzugs auf einer Zahnoberfläche zum Schützen einer gesunden Zahnoberfläche gegenüber einer Erosion durch eine saure Ätzlösung bei einer Zahnreparaturbehandlung, dadurch gekennzeichnet, daß das Polymere eine Mischung aus einem Copolymeren (A) aus Vinylchlorid und Vinylacetat und einem Copolymeren (B) aus Vinylchlorid, Vinylacetat und einer Vinylverbindung mit einer Carbonsäure oder Carbonsäureanhydridgruppe oder -gruppen aufweist, wobei das Molverhältnis von Vinylchlorid zu Vinylacetat in den Copolymeren (A) und (B) (95 bis 60):(5 bis 40) beträgt und die Molmenge der Vinylverbindung mit einer Carbonsäure- oder Carbonsäureanhydridgruppe oder -gruppen zu der Summe des Vinylchlorids und des Vinylacetats in den Copolymeren (B) 1 bis 20% beträgt.

2. Lack nach Anspruch 1, in welchem das Gewichtsverhältnis des Copolymeren (A) zu dem Copolymeren (B) im Bereich von (95 bis 40):(5 bis 60) liegt.

3. Lack nach Anspruch 2, wobei das Verhältnis im Bereich von (90 bis 60):(10 bis 40) liegt.

4. Lack nach einem der Ansprüche 1 bis 3, wobei das Molverhältnis von Vinylchlorid zu Vinylacetat in den Copolymeren (A) und (B) (90 bis 72):(10 bis 28) beträgt.

5. Lack nach einem der Ansprüche 1 bis 4, welcher ausserdem 3 bis 10 Gew.-% (bezogen auf das Polymer) eines Silankupplungsmittels enthält, um das Kleben der die Zahnoberfläche schützenden Komponenten an der Zahnoberfläche zu erhöhen.

6. Lack nach einem der Ansprüche 1 bis 5, welcher ausserdem 0,01 bis 2 Gew.-% (bezogen auf die Lösung) eines Farbstoffes enthält, um die Grenze zwischen dem Teil der zu schützenden Zahnoberfläche und dem zu reparierenden Teil zu kennzeichnen.

7. Lack nach einem der Ansprüche 1 bis 6, wobei die Konzentration der gesamten Copolymeren 1 bis 20 Gew.-%, bezogen auf die Lösung, beträgt.

8. Lack nach einem der Ansprüche 1 bis 7, wobei das Lösungsmittel aus Aceton, Diethylether, Diisopropylether, Ethanol, Ethylacetat, n-Propylacetat, Isopropylacetat, n-Butylacetat, Isobutylacetat, Methylenchlorid, Ethylenchlorid oder Chlorform oder einer Mischung aus zwei oder mehreren derartigen Verbindungen besteht.

9. Lack nach einem der Ansprüche 1 bis 8, wobei jedes der Copolymeren (A) und (B) ein Molekulargewicht im Bereich von 20000 bis 70000 besitzt.

## Revendications

1. Vernis comprenant un polymère qui a le chlorure de vinyle et l'acétate de vinyle comme motifs constitutifs principaux et qui est dissous dans un solvant, le vernis convenant pour l'utilisation dans la formation d'un revêtement sur la surface des dents pour protéger la surface saine des dents contre l'érosion par une solution de décapage acide dans le traitement de reconstitution des dents, caractérisé en ce que le polymère comprend un mélange d'un copolymère (A) de chlorure de vinyle et d'acétate de vinyle et d'un copolymère (B) de chlorure de vinyle, d'acétate de vinyle et d'un composé vinylique ayant un ou plusieurs groupes acides carboxylique ou anhydride carboxylique, le rapport molaire du chlorure de vinyle à l'acétate de vinyle dans les copolymères (A) et (B) étant de (95 à 60):(5 à 40) et la proportion molaire du composé vinylique ayant un ou plusieurs groupes acide carboxylique ou anhydride carboxylique par rapport à la somme du chlorure de vinyle et de l'acétate de vinyle dans le copolymère (B) étant de 1 à 20%.

2. Vernis suivant la revendication 1, dans lequel la proportion pondérale du copolymère (A) au copolymère (B) est dans l'intervalle de (95 à 40):(5 à 60).

3. Vernis suivant la revendication 2, dans lequel cette proportion est dans l'intervalle de (90 à 60):(10 à 40).

4. Vernis suivant l'une quelconque des revendications 1 à 3, dans lequel le rapport molaire du chlorure de vinyle à l'acétate de vinyle dans les copolymères (A) et (B) est de (90 à 72):(10 à 28).

5. Vernis suivant l'une quelconque des revendications 1 à 4, qui contient en outre 3 à 10% (par rapport au polymère) d'un silane comme agent de couplage de manière à augmenter le caractère collant des constituants protégeant la surface des dents à la surface des dents.

6. Vernis suivant l'une quelconque des revendications 1 à 5, qui contient en outre 0,01 à 2% en poids (par rapport à la solution) d'un colorant pour indiquer la limite entre la partie de la surface des dents à protéger et la partie de celles-ci à reconstituer.

7. Vernis suivant l'une quelconque des revendications 1 à 6, dans lequel la concentration des copolymères totaux est de 1 à 20% en poids par rapport à la solution.

8. Vernis suivant l'une quelconque des revendications 1 à 7, dans lequel le solvant est l'acétone, l'éther diéthylique, l'éther diisopropylique, l'éthanol, l'acétate d'éthyle, l'acétate de n-propyle, l'acétate d'isopropyle, l'acétate de n-butyle, l'acétate d'isobutyle, le chlorure de méthylène, le chlorure d'éthylène ou le chloroforme ou un mélange de plusieurs de ces composés.

9. Vernis suivant l'une quelconque des revendications 1 à 8, dans lequel chacun des copolymères (A) et (B) a une masse moléculaire dans l'intervalle de 20000 à 70000.